Europäisches Patentamt

**European Patent Office**

Office européen des brevets

(19)

(11) Veröffentlichungsnummer: **0 044 050**
**B1**

(12) EUROPÄISCHE PATENTSCHRIFT

(45) Veröffentlichungstag der Patentschrift:
**13.02.85**

(21) Anmeldenummer: **81105368.5**

(22) Anmeldetag: **10.07.81**

(51) Int. Cl.⁴: **C 08 B 37/02,** A 61 K 33/26,
A 61 K 31/715

(54) **Verfahren zur Herstellung von Eisen (III)-Hydroxid-Dextran-Komplexen und diese enthaltende pharmazeutische sterile Lösung.**

(30) Priorität: **16.07.80 DE 3026868**

(43) Veröffentlichungstag der Anmeldung:
**20.01.82 Patentblatt 82/3**

(45) Bekanntmachung des Hinweises auf die Patenterteilung:
**13.02.85 Patentblatt 85/7**

(84) Benannte Vertragsstaaten:
**AT BE CH FR GB IT LI NL SE**

(56) Entgegenhaltungen:
**DE - B - 1 172 250**
**DE - B - 1 196 629**
**FR - A - 2 150 794**
**GB - A - 748 024**
**US - A - 4 180 567**

(73) Patentinhaber: **Laboratorien Hausmann AG,**
**Rechenstrasse 37, CH-9001 St. Gallen (CH)**

(72) Erfinder: **Richle, Walter, Dr., Weirden 21,**
**CH-9062 Lustmühle (Teufen) (CH)**
Erfinder: **Müller, Arthur, Dr., Schoeckstrasse 10,**
**CH-9008 St. Gallen (CH)**

(74) Vertreter: **Türk, Dietmar, Dr. rer. nat. et al, Redies,**
**Redies, Türk & Gille Patentanwälte Brucknerstrasse 20,**
**D-4000 Düsseldorf 13 (DE)**

EP 0 044 050 B1

# 0 044 050

## Beschreibung

Verfahren zur Herstellung von Eisen(III)-Hydroxid-Dextran-Komplexen und diese enthaltende pharmazeutische sterile Lösung.

Die vorliegende Erfindung betrifft ein Verfahren zur Herstellung von Eisen(III)-Hydroxid-Dextran-Komplexen, wobei zu einer sauren, ein teilweise depolymerisiertes Dextran und ein Eisen(III)-Salz enthaltenden Lösung ein Alkalicarbonat, Ammoniumcarbonat oder ein Carbonat einer gegenüber den Reaktionskomponenten inerten organischen Base und anschließend ein Alkalimetallhydroxid oder Ammoniumhydroxid zugegeben wird, die gebildete Suspension durch Erhitzen in Lösung gebracht und die Lösung in an sich bekannter Weise aufgearbeitet wird.

Verfahren zur Herstellung von nichtionischen, therapeutisch verwertbaren Eisen(III)-Hydroxid-Dextran-Komplexen sind in großer Anzahl bekannt. Das eingangs geschilderte Verfahren ist z. B. in der DE-PS 1 196 629 beschrieben und führt zu vorzüglichen Präparaten, die mit großem Erfolg zur Bekämpfung von Eisenmangelzuständen bei Mensch und Tier eingesetzt wurden. Die Lösungen dieser Komplexe für Injektionszwecke enthielten jedoch nur bis zu etwa 10% Eisen (G./V.). (»G« bedeutet »Gewicht« und »V« bedeutet »Volumen«). Es besteht ein großes Bedürfnis, Eisen-Dextran-Komplexe zu schaffen, aus denen injizierbare Lösungen mit höherem Eisengehalt erhalten werden können.

Aus den US-PS 3 536 696 und 3 639 588 sind Eisen(III)-Hydroxid-Komplexe bekannt, die als Komplexbildner Dextranheptonsäure enthalten. Die Herstellung der Dextranheptonsäure ist umständlich, und das Gesamtverfahren ist deshalb nicht sehr befriedigend.

Aus der DE-OS 1 768 361 ist ein Verfahren zur Herstellung von Eisen(III)-Hydroxid-Komplexen bekannt, wobei man eine wäßrige Lösung aus einem Eisen(III)-Salz langsam mit Alkali neutralisiert, dann eine wäßrige Lösung eines Dextrans zufügt, den pH-Wert der Lösung auf 4 bis 7 einstellt und die Lösung dann zur Bildung des Komplexes erhitzt. Aus diesen Komplexen sollen sich injizierbare Lösungen mit einem Gehalt von bis zu 25 Gew.-% Eisen bezogen auf das Volumen der Lösung herstellen lassen. Der Nachteil dieses Verfahrens liegt darin, daß nur bestimmte (oxydierte) Dextrane bei pH 4 bis 7 zur Lösung führen, während für andere Dextrane (nicht oxydierte) höhere pH-Werte als 7 notwendig sind, um die beschriebenen Eisen-Dextran-Komplexe zu erhalten.

Aus der US-PS 4 180 567 ist ein Verfahren zur Herstellung von Dextran-Eisen-Komplexen bekannt, gemäß dem das Dextran oder eine andere Polyhydroxy-Verbindung mit einer Base bei einer Temperatur von 85 bis 100° C vorbehandelt wird. Die auf diese Weise »aktivierte« Polyhydroxy-Verbindung wird dann mit einer wäßrigen Lösung einer Eisenverbindung vermischt, die im wesentlichen dialysiertes Eisen(III)-Hydroxid enthält. Dieses Verfahren hat den Nachteil, daß in zwei getrennten Operationen Vorrichtungen zunächst die aktivierte Dextranlösung und daneben die Lösung enthaltend das dialysierte Eisen(III)-Hydroxid hergestellt werden müssen. Nach diesem Verfahren resultieren nur Eisen-Dextran-Komplexe mit 50—150 mg Fe/ml, d. h. mit 5—15% G/V Eisengehalt.

Der vorliegenden Erfindung liegt die Aufgabenstellung zugrunde, in einfacher und sicherer Weise Eisen(III)-Hydroxid-Dextran-Komplexe herzustellen, die stabile sterile Lösungen für Injektionszwecke mit einem hohen Gehalt von über 20% G/V Eisen der Lösung ergeben.

Gegenstand der vorliegenden Erfindung ist demgemäß ein Verfahren zur Herstellung von Eisen(III)-Hydroxid-Dextran-Komplexen, wobei zu einer sauren, ein teilweise depolymerisiertes Dextran und ein Eisen(III)-Salz enthaltenden Lösung ein Alkalicarbonat, Ammoniumcarbonat oder ein Carbonat einer gegenüber den Reaktionskomponenten inerten organischen Base und anschließend ein Alkalimetallhydroxid oder Ammoniumhydroxid zugegeben wird, die gebildete Suspension durch Erhitzen in Lösung gebracht und die Lösung in an sich bekannter Weise aufgearbeitet wird, das dadurch gekennzeichnet ist, daß man

a) als Dextran ein solches mit einem mittleren Molekulargewicht von 1000 bis 10 000 verwendet,
b) auf 100 kg Dextran 50 bis 100 kg Eisenionen einsetzt,
c) zu der Lösung aus Dextran und Eisen(III)-Salz 2,0 bis 2,6 Val Carbonat bezogen auf die Eisen(III)-Ionen während mehr als 2 Stunden zugibt,
d) dann zu der Lösung Alkalimetallhydroxid oder Ammoniumhydroxid in einer solchen Menge zugibt, daß einschließlich des zugegebenen Carbonats etwa 3 Val Anionen zugefügt sind, bis ein pH-Wert nicht kleiner als etwa 10,5 erreicht ist.

Das Verfahren gemäß der Erfindung läßt sich in überraschend einfacher Weise in einer Vorrichtung durchführen, d. h. als sogenanntes Eintopfverfahren, wobei lediglich in einem gesonderten Gefäß die Lösungen der Reaktionskomponenten hergestellt werden müssen.

Als geeignete Dextrane werden gemäß der Erfindung vorzugsweise Dextrane mit einem mittleren Molekulargewicht von mindestens etwa 3000, bevorzugt mindestens etwa 4000 verwendet. Die obere Grenze für das Molekulargewicht liegt zweckmäßig bei etwa 7000, vorzugsweise bei etwa 6000. Besonders gute Ergebnisse wurden mit Dextranen mit einem mittleren Molekulargewicht von etwa 4000—6000 erzielt. Als mittleres Molekulargewicht wird das Gewichtsmittel der eingesetzten Dextranfraktion verstanden.

Bevorzugt wird eine Dextranlösung verwendet, die mindestens 20, vorzugsweise mindestens 30 kg

2

Dextran in 100 l Wasser gelöst enthält. Die obere Grenze für die Konzentration liegt zweckmäßig bei 80 kg, vorzugsweise bei 60 kg Dextran, gelöst in 100 l Wasser.

Als Eisenlösungen werden zweckmäßig solche verwendet, die mindestens etwa 3, vorzugsweise mindestens 5% Eisen(III)-Ionen (G./G) enthalten. Die obere Grenze für die Konzentration an Eisen(III)-Ionen liegt zweckmäßig bei etwa 10%, bevorzugt bei etwa 8% (G./G).

Als Eisen(III)-Salze werden gemäß dem Stand der Technik übliche Salze verwendet, wie Eisen(III)-chlorid, -nitrat, -acetat, -sulfat und andere chemische Äquivalente. Besonders bevorzugt ist Eisen(III)-chlorid.

Die Lösung des Dextrans in Wasser wird zweckmäßig durch Erwärmen, z. B. auf 60 bis 80° C erzeugt. Nach einer bevorzugten Ausführungsform der Erfindung wird die abgekühlte Lösung des Dextrans in die Lösung des Eisen(III)-Salzes einlaufen gelassen. Ein Erwärmen ist also nicht erforderlich, und beide Lösungen haben eine Temperatur von zweckmäßig weniger als 40° C, vorzugsweise etwa Raumtemperatur.

Der Lösung aus Dextran und Eisen(III)-Salz wird dann ein Alkalicarbonat, Ammoniumcarbonat oder ein Carbonat einer gegenüber den Reaktionskomponenten inerten organischen Base in einer Menge von 2,0 bis 2,6 Val bezogen auf das Eisen(III)-Ionen langsam zudosiert. Wenn Eisen(III)-Chlorid als Eisen(III)-Salz eingesetzt wird, sind nach dieser Zugabe also etwa 2,0 bis 2,6 Chlorionen durch Hydroxylionen ersetzt. Die Zugabe des Carbonats, zweckmäßig in einer wäßrigen Lösung, erfolgt zweckmäßig ebenfalls bei einer Temperatur von weniger als 40°C, vorzugsweise ohne Erwärmen, d. h. bei Raumtemperatur. Die Lösung wird vorzugsweise sehr langsam während mehr als 2 Stunden, besonders bevorzugt mehr als 3 Stunden zugegeben. Die Carbonatlösung soll nicht zu verdünnt sein, um große Flüssigkeitsmengen zu vermeiden. Wenn Natriumcarbonat verwendet wird, was u. a. aus Kostengründen bevorzugt ist, haben sich Lösungen mit einer Konzentration von etwa 15 bis 20% (G./G) insbesondere etwa 17 bis 18% als geeignet erwiesen. Nach der Zugabe liegt der pH-Wert der Lösung bei etwa 1,5—2,0, vorzugsweise bei etwa 1,7 bis 1,8.

Danach wird ein Alkalimetallhydroxid oder Ammoniumhydroxid zugegeben. Auch dies erfolgt zweckmäßig ohne Erwärmen, d. h. unter den gleichen Bedingungen, wie die Zugabe des Carbonats. Bevorzugt ist, u. a. auch wieder aus Kostengründen, Natronlauge, wobei Konzentrationen von etwa 20 bis 40%, vorzugsweise etwa 25 bis 35% (G./G) geeignet sind. Die Menge der Base ist so groß, daß im wesentlichen alle Anionen des Eisen(III)-Salzes durch Hydroxyl-Ionen ersetzt werden. Um dies zu erreichen, ist ein geringer Überschuß von Base zweckmäßig, der nicht mehr als 5%, bevorzugt nicht mehr als 2%, bezogen auf die Zahl der positiven Ladungen der vorhandenen Eisen(III)-Ionen betragen sollte. Die Zugabe der Base während verhältnismäßig kurzer Zeit, vorzugsweise während 15 bis 45 Min., besonders bevorzugt während etwa 25 bis 35 Min.

Nach der Zugabe der Base beträgt der pH-Wert der Lösung mindestens etwa 10,5 jedoch zweckmäßig nicht mehr als 12. Vorzugsweise liegt er bei etwa 11,0 bis 11,5 oder besser bei 11,2 bis 11,4.

Der Abschluß der Bildung der Suspension kann dadurch festgestellt werden, daß eine Probe der Reaktionslösung zentrifugiert wird. Wenn nach Zusatz von weiterem Alkalimetallhydroxid zur klaren überstehenden Lösung keine weitere Fällung mehr auftritt, kann die Bildung der Suspension als beendet angesehen werden.

Anschließend wird das Reaktionsgemisch erhitzt, bis die suspendierten Anteile gelöst sind. Es wird dazu zweckmäßig möglichst schnell auf hohe Temperaturen von mindestens etwa 80°C bis zum Siedepunkt, zweckmäßig einfach bis zum Siedepunkt erhitzt. Bei dieser Temperatur wird die Lösung etwa 40 Min. bis 2 Stunden, vorzugsweise etwa 50 bis 80 Min. gehalten.

Die Aufarbeitung der so erhaltenen Lösung des Eisen(III)-Hydroxid-Dextran-Komplexes kann in an sich bekannter Weise erfolgen. Sie kann vor der Reinigung und Isolierung durch Zugabe einer festen, flüssigen oder gasförmigen Säure, wie Kationenaustauscher in der H-Form, Schwefelsäure oder Chlorwasserstoffsäure neutralisiert werden. Um unerwünscht hohe Elektrolytkonzentrationen in der Lösung zu beseitigen, kann zusätzlich zum Kationenaustauscher noch ein Anionenaustauscher in HO-Form zugegeben oder die Lösung gegen Wasser dialysiert werden. Gut wasserlösliche Festpräparate können aus den Lösungen der neuen Eisenkomplexe durch die bekannten Verfahren, wie beispielsweise Eindampfen der neutralen Lösungen unter vermindertem Druck oder durch fraktionierte Fällung mit einem wassermischbaren organischen Lösungsmittel wie z. B. Methanol, Äthanol oder Aceton erhalten werden.

Nach einer bevorzugten Ausführungsform wird nach Abkühlen auf unter etwa 30° C bis Raumtemperatur mit verdünnter Salzsäure der pH-Wert schwach sauer, z. B. auf etwa 5 bis 6, zweckmäßig etwa 5,5 eingestellt. Nach Durchlauf durch einen Klärseparator (z. B. Tellerzentrifuge) zwecks Abtrennung von gröberen Verunreinigungen und Filtration der Lösung kann der Komplex durch Zugabe eines geeigneten mit Wasser mischbaren Lösungsmittels gefällt werden. Wie nach dem Stand der Technik ist hierbei Äthanol bevorzugt. Der Niederschlag wird abgetrennt und getrocknet und enthält etwa 28—35 Gew.-% Fe.

Es ist überraschenderweise möglich, den gemäß dem Verfahren der Erfindung hergestellten Komplex in hoher Konzentration in Wasser lösen zu können, z. B. in einer Konzentration von 15 bis 25% Fe, vorzugsweise etwa 20% Fe oder darüber (G./V.).

Gegenstand der Erfindung sind weiterhin pharmazeutische sterile wäßrige Lösungen zur Behandlung von Eisenmangelzuständen, enthaltend den gemäß dem vorstehend beschriebenen Verfahren erhältlichen Eisen(III)-Hydroxid-Dextran-Komplex und übliche Zusatzstoffe. Zur Herstellung einer solchen Lösung wird der Komplex in Wasser durch Erwärmen, z. B. auf Temperaturen von etwa 60 bis 80° C, gelöst. Anschließend wird sterilisiert. Übliche Zusatzstoffe für die sterilisierte wäßrige Lösung sind z. B. 0,5% Phenol.

Beispiel

80 kg Dextran (Molekulargewicht 5000) werden in 180 l Wasser von 70° C gelöst. Die auf Raumtemperatur abgekühlte Lösung läßt man in 877 kg wäßrige $FeCl_3$-Lösung (6,4% G/G Fe, Dichte (20° C) = 1,170) einlaufen. Dieser gemischten Lösung werden bei 25° C 751 kg wäßrige Sodalösung (17,2% G/G $Na_2CO_3$, Dichte (20° C) = 1,185) innerhalb 3,5 Stunden unter mechanischem Rühren zudosiert. Dadurch wird erreicht, daß im $FeCl_3$ etwa 2,3 $Cl^-$ durch $OH^-$ (entsprechend $Fe(OH)_{2,3}Cl_{0,7}$) ersetzt werden. Der pH der Reaktionslösung erreicht dabei den Wert von etwa 1,7. Nach Zugabe von 93 kg Natronlauge (30% G/G NaOH, Dichte (20° C) = 1,330) inert etwa 30 Minuten resultiert ein pH von etwa 11. Die dabei entstandene Suspension wird zum Sieden erhitzt und während etwa 1 Stunde bei dieser Temperatur gehalten. Die resultierende Eisen(III)-Hydroxid-Dextran-Komplex-Lösung wird auf 25° C abgekühlt und mit 21 kg verdünnter Salzsäure (20,4% G/G HCl, Dichte (20° C) = 1,100) auf pH 5,5 eingestellt. Nach Durchlauf durch einen Klärseparator (Tellerzentrifuge) zwecks Abtrennung von gröberen Verunreinigungen und Filtration der Lösung durch ein Mehrschichtenfilter wird mit Äthanol gefällt. Der Niederschlag wird in einem Mixer mit Äthanol gewaschen, abgetrennt und im Vakuum getrocknet. Der Eisengehalt des Trockenmaterials beträgt 28—35% G/G Fe. Der trockene Eisenkomplex wird in destilliertem, pyrogenfreiem Wasser bei 70° C zu einer 20% G/V eisenhaltigen sterilen Injektionslösung verarbeitet, die 0,5% V/V Phenol als Konservierungsmittel enthält. Die relative Viskosität dieser Lösung bei 25° C ist kleiner als 40.

**Patentansprüche für die Vertragsstaaten: BE, GB, FR, NL, SE, CH, IT, LI**

1. Verfahren zur Herstellung von Eisen(III)-Hydroxid-Dextran-Komplexen, wobei zu einer sauren, ein teilweise depolymerisiertes Dextran und ein Eisen(III)-Salz enthaltenden Lösung ein Alkalicarbonat, Ammoniumcarbonat oder ein Carbonat einer gegenüber den Reaktionskomponenten inerten organischen Base und anschließend ein Alkalimetallhydroxid oder Ammoniumhydroxid zugegeben wird, die gebildete Suspension durch Erhitzen in Lösung gebracht und die Lösung in an sich bekannter Weise aufgearbeitet wird, dadurch gekennzeichnet, daß man

    a)    als Dextran ein solches mit einem mittleren Molekulargewicht von 1000 bis 10 000 verwendet,
    b)    auf 100 kg Dextran 50 bis 100 kg Eisenionen einsetzt,
    c)    zu der Lösung aus Dextran und Eisen(III)-Salz 2,0 bis 2,6 Val Carbonat, bezogen auf die Eisen(III)-Ionen, während mehr als 2 Stunden zugibt,
    d)    dann zu der Lösung Alkalimetallhydroxid oder Ammoniumhydroxid in einer solchen Menge zugibt, daß einschließlich des zugegebenen Carbonats etwa 3 Val Anionen zugefügt sind, bis ein pH-Wert nicht kleiner als etwa 10,5 erreicht ist.

2. Verfahren gemäß Anspruch 1, dadurch gekennzeichnet, daß das Dextran ein Molekulargewicht von 3000 bis 7000, vorzugsweise 4000 bis 6000 hat.
3. Verfahren gemäß einem der Ansprüche 1 bis 3, dadurch gekennzeichnet, daß man die abgekühlte Lösung des Dextrans in die Lösung des Eisen(III)-Salzes einlaufen läßt.
4. Verfahren gemäß einem der Ansprüche 1 bis 4, dadurch gekennzeichnet, daß man die Lösung des Dextrans bei weniger als 40° C in die Lösung des Eisen(III)-Salzes einlaufen läßt.
5. Verfahren gemäß einem der Ansprüche 1 bis 5, dadurch gekennzeichnet, daß man das Carbonat bei weniger als 40° C zufügt.
6. Verfahren gemäß einem der Ansprüche 1 bis 6, dadurch gekennzeichnet, daß man eine wäßrige Lösung des Carbonats sehr langsam während mehr als 2 Stunden, vorzugsweise mehr als 3 Stunden zugibt.
7. Verfahren gemäß einem der Ansprüche 1 bis 7, dadurch gekennzeichnet, daß man die Lösung des Alkalimetallhydroxids oder Ammoniumhydroxids während 15 bis 45 Min. zugibt.
8. Pharmazeutische, sterile, wäßrige Lösung zur Behandlung von Eisenmangelzuständen enthaltend den gemäß Anspruch 1 bis 8 erhältlichen Eisen(III)-Hydroxid-Dextran-Komplex und übliche Zusatzstoffe.

## 0 044 050

### Patentansprüche für den Vertragsstaat: AT

1. Verfahren zur Herstellung von Eisen(III)-Hydroxid-Dextran-Komplexen, wobei zu einer sauren, ein teilweise depolymerisiertes Dextran und ein Eisen(III)-Salz enthaltenden Lösung ein Alkalicarbonat, Ammoniumcarbonat oder ein Carbonat einer gegenüber den Reaktionskomponenten inerten organischen Base und anschließend ein Alkalimetallhydroxid oder Ammoniumhydroxid zugegeben wird, die gebildete Suspension durch Erhitzen in Lösung gebracht und die Lösung in an sich bekannter Weise aufgearbeitet wird, dadurch gekennzeichnet, daß man

   a)   als Dextran ein solches mit einem mittleren Molekulargewicht von 1000 bis 10 000 verwendet,
   b)   auf 100 kg Dextran 50 bis 100 kg Eisenionen einsetzt,
   c)   zu der Lösung aus Dextran und Eisen(III)-Salz 2,0 bis 2,6 Val Carbonat, bezogen auf die Eisen(III)-Ionen, während mehr als 2 Stunden zugibt,
   d)   dann zu der Lösung Alkalimetallhydroxid oder Ammoniumhydroxid in einer solchen Menge zugibt, daß einschließlich des zugegebenen Carbonats etwa 3 Val Anionen zugefügt sind, bis ein pH-Wert nicht kleiner als etwa 10,5 erreicht ist.

2. Verfahren gemäß Anspruch 1, dadurch gekennzeichnet, daß das Dextran ein Molekulargewicht von 3000 bis 7000, vorzugsweise 4000 bis 6000 hat.

3. Verfahren gemäß einem der Ansprüche 1 bis 3, dadurch gekennzeichnet, daß man die abgekühlte Lösung des Dextrans in die Lösung des Eisen(III)-Salzes einlaufen läßt.

4. Verfahren gemäß einem der Ansprüche 1 bis 4, dadurch gekennzeichnet, daß man die Lösung des Dextrans bei weniger als 40°C in die Lösung des Eisen(III)-Salzes einlaufen läßt.

5. Verfahren gemäß einem der Ansprüche 1 bis 5, dadurch gekennzeichnet, daß man das Carbonat bei weniger als 40°C zufügt.

6. Verfahren gemäß einem der Ansprüche 1 bis 6, dadurch gekennzeichnet, daß man eine wäßrige Lösung des Carbonats sehr langsam während mehr als 2 Stunden, vorzugsweise mehr als 3 Stunden zugibt.

7. Verfahren gemäß einem der Ansprüche 1 bis 7, dadurch gekennzeichnet, daß man die Lösung des Alkalimetallhydroxids oder Ammoniumhydroxids während 15 bis 45 Min. zugibt.

### Claims for the Contracting States: BE, GB, FR, NL, SE, CH, IT, LI

1. A process for the production of iron-(III) hydroxide/dextran complexes in which an alkali carbonate, ammonium carbonate or a carbonate of an organic base inert to the reaction components and then an alkali metal hydroxide or ammonium hydroxide are added to an acidic solution containing a partially depolymerized dextran and an iron-(III) salt, the suspension formed is converted into a solution by heating and the solution is worked up in known manner, characterized in that

   a)   the dextran used is one having an average molecular weight of from 1000 to 10 000,
   b)   from 50 to 100 kg of iron ions are used per 100 kg of dextran,
   c)   from 2.0 to 2.6 vals of carbonate, based on the iron-(III) ions, are added to the solution of dextran and iron-(III) salt over a period of more than 2 hours,
   d)   alkali metal hydroxide or ammonium hydroxide is then added to the solution in such a quantity that approximately 3 vals of anions, including the carbonate added, are added until a pH-value of no lower than about 10.5 is reached.

2. A process as claimed in Claim 1, characterized in that the dextran has a molecular weight of from 3000 to 7000 and preferably from 4000 to 6000.

3. A process as claimed in any of Claims 1 to 3, characterized in that the cooled solution of the dextran is run into the solution of the iron-(III) salt.

4. A process as claimed in any of Claims 1 to 4, characterized in that the solution of the dextran is run into the solution of the iron-(III) salt at a temperature below 40°C.

5. A process as claimed in any of Claims 1 to 5, characterized in that the carbonate is added at a temperature below 40°C.

6. A process as claimed in any of Claims 1 to 6, characterized in that an aqueous solution of the carbonate is added very slowly over a period of more than 2 hours and preferably over a period of more than 3 hours.

7. A process as claimed in any of Claims 1 to 7, characterized in that the solution of the alkali metal hydroxide or ammonium hydroxide is added over a period of from 15 to 45 minutes.

8. A pharmaceutical sterile aqueous solution for treating iron deficiency conditions containing the iron-(III) hydroxide/dextran complex obtainable by the process claimed in Claims 1 to 8 and the usual additives.

5

**Claims for the Contracting State: AT**

1. A process for the production of iron-(III) hydroxide/dextran complexes in which an alkali carbonate, ammonium carbonate or a carbonate of an organic base inert to the reaction components and then an alkali metal hydroxide or ammonium hydroxide are added to an acidic solution containing a partially depolymerized dextran and an iron-(III) salt, the suspension formed is converted into a solution by heating and the solution is worked up in known manner, characterized in that

   a) the dextran used is one having an average molecular weight of from 1000 to 10 000,
   b) from 50 to 100 kg of iron ions are used per 100 kg of dextran,
   c) from 2.0 to 2.6 vals of carbonate, based on the iron-(III) ions, are added to the solution of dextran and iron-(III) salt over a period of more than 2 hours,
   d) alkali metal hydroxide or ammonium hydroxide is then added to the solution in such a quantity that approximately 3 vals of anions, including the carbonate added, are added until a pH-value of no lower than about 10.5 is reached.

2. A process as claimed in Claim 1, characterized in that the dextran has a molecular weight of from 3000 to 7000 and preferably from 4000 to 6000.

3. A process as claimed in any of Claims 1 to 3, characterized in that the cooled solution of the dextran is run into the solution of the iron-(III) salt.

4. A process as claimed in any of Claims 1 to 4, characterized in that the solution of the dextran is run into the solution of the iron-(III) salt at a temperature below 40° C.

5. A process as claimed in any of Claims 1 to 5, characterized in that the carbonate is added at a temperature below 40° C.

6. A process as claimed in any of Claims 1 to 6, characterized in that an aqueous solution of the carbonate is added very slowly over a period of more than 2 hours and preferably over a period of more than 3 hours.

7. A process as claimed in any of Claims 1 to 7, characterized in that the solution of the alkali metal hydroxide or ammonium hydroxide is added over a period of from 15 to 45 minutes.

**Revendications pour les Etats contractants: BE, GB, FR, NL, SE, CH, IT, LI**

1. Procédé de préparation de complexes hydroxyde de fer(III)-dextran où l'on ajoute à une solution acide contenant un dextran partiellement dépolymérisé et un sel de fer(III) un carbonate alcalin, un carbonate d'ammonium ou un carbonate d'une base organique inerte vis-à-vis des composants de la réaction puis un hydroxyde de métal alcalin ou de l'hydroxyde d'ammonium, en ce qu'on met en solution la suspension formée en chauffant et en ce qu'on traite la solution de façon connue, caractérisé en ce que

   a) on utilise comme dextran un dextran ayant un poids moléculaire moyen de 1000 à 10 000,
   b) on utilise sur 100 kg de dextran 50 à 100 kg d'ions fer,
   c) on ajoute à la solution de dextran et de sel de fer(III) de 2,0 à 2,6 Val de carbonate par rapport aux ions fer(III), pendant plus de 2 h,
   d) puis on ajoute à la solution un hydroxyde de métal alcalin ou de l'hydroxyde d'ammonium en une quantité telle qu'y compris le carbonate ajouté on ajoute environ 3 Val d'anions, jusqu'à ce qu'on atteigne un pH d'au moins environ 10,5.

2. Procédé selon la revendication 1, caractérisé en ce que le dextran a un poids moléculaire de 3000 à 7000, de préférence de 4000 à 6000.

3. Procédé selon l'une des revendications 1 à 3, caractérisé en ce qu'on introduit la solution refroidie du dextran dans la solution du sel de fer(III).

4. Procédé selon l'une des revendications 1 à 4, caractérisé en ce qu'on introduit la solution du dextran à moins de 40° C dans la solution du sel de fer(III).

5. Procédé selon l'une des revendications 1 à 5, caractérisé en ce qu'on ajoute le carbonate à moins de 40° C.

6. Procédé selon l'une des revendications 1 à 6, caractérisé en ce qu'on ajoute une solution aqueuse du carbonate très lentement pendant plus de 2 h, de préférence pendant plus de 3 h.

7. Procédé selon l'une des revendications 1 à 7, caractérisé en ce qu'on ajoute la solution de l'hydroxyde de métal alcalin ou de l'hydroxyde d'ammonium pendant 15 à 45 minutes.

8. Solution pharmaceutique aqueuse stérile pour le traitement des états de déficience en fer contenant le complexe hydroxyde de fer(III)-dextran selon les revendications 1 à 8 et les additifs habituels.

# 0 044 050

**Revendications pour l'Etat contractant: AT**

1. Procédé de préparation de complexes hydroxyde de fer(III)-dextran où l'on ajoute à une solution acide contenant un dextran partiellement dépolymérisé et un sel de fer(III) un carbonate alcalin, un carbonate d'ammonium ou un carbonate d'une base organique inerte vis-à-vis des composants de la réaction puis un hydroxyde de métal alcalin ou de l'hydroxyde d'ammonium, en ce qu'on met en solution la suspension formée en chauffant et en ce qu'on traite la solution de façon connue, caractérisé en ce que

    a)   on utilise comme dextran un dextran ayant un poids moléculaire moyen de 1000 à 10 000,
    b)   on utilise sur 100 kg de dextran 50 à 100 kg d'ions fer,
    c)   on ajoute à la solution de dextran et de sel de fer(III) de 2,0 à 2,6 Val de carbonate par rapport aux ions fer(III), pendant plus de 2 h,
    d)   puis on ajoute à la solution un hydroxyde de métal alcalin ou de l'hydroxyde d'ammonium en une quantité telle qu'y compris le carbonate ajouté on ajoute environ 3 Val d'anions, jusqu'à ce qu'on atteigne un pH d'au moins environ 10,5.

2. Procédé selon la revendication 1, caractérisé en ce que le dextran a un poids moléculaire de 3000 à 7000, de préférence de 4000 à 6000.

3. Procédé selon l'une des revendications 1 à 3, caractérisé en ce qu'on introduit la solution refroidie du dextran dans la solution du sel de fer(III).

4. Procédé selon l'une des revendications 1 à 4, caractérisé en ce qu'on introduit la solution du dextran à moins de 40°C dans la solution du sel de fer(III).

5. Procédé selon l'une des revendications 1 à 5, caractérisé en ce qu'on ajoute le carbonate à moins de 40°C.

6. Procédé selon l'une des revendications 1 à 6, caractérisé en ce qu'on ajoute une solution aqueuse du carbonate très lentement pendant plus de 2 h, de préférence pendant plus de 3 h.

7. Procédé selon l'une des revendications 1 à 7, caractérisé en ce qu'on ajoute la solution de l'hydroxyde de métal alcalin ou de l'hydroxyde d'ammonium pendant 15 à 45 minutes.

7